# FASCICULE DE BREVET EUROPEEN

(11) **EP 0 828 467 B1**
(45) Date de publication et mention de la délivrance du brevet: **27.10.1999**
(21) Numéro de dépôt: 96906820.4
(22) Date de dépôt: 12.03.1996
(51) Int. Cl.: A61F 13/15

(54) **SYSTEME PROTECTION TOTALE POUR COUCHES, SERVIETTES PERIODIQUES**
VOLLSTÄNDIGER SCHUTZ IN WINDELN UND DAMENBINDEN
TOTAL PROTECTION SYSTEM FOR DIAPERS AND SANITARY NAPKINS

(30) Priorité: 26.04.1995 FR 9504985
(43) Date de publication de la demande: 18.03.1998
(73) Titulaire: Capriulo, Claudio, 60160 Montataire (FR); Eveillard, Eliane, 77390 Verneuil-l'Etang (FR)
(72) Inventeur: Capriulo, Claudio, 60160 Montataire (FR); Eveillard, Eliane, 77390 Verneuil-l'Etang (FR)
(86) Numéro de dépôt international: FR9600385
(87) Numéro de publication internationale: WO9633681

(56) Documents cités:
- WO-A-91/09582
- WO-A-93/12745

## Description

La présente invention concerne un procédé de fabrication d'un système de protection totale applicable à toutes sortes de couches pour adultes incontinents, bébés ou enfants et serviettes périodiques.

Les systèmes de fabrication actuels,(exemple,brevet WO-A-91/09582) basés sur la superposition de couches absorbantes, recouvertes d'un voile de non tissé anallergique reposant sur un film de polyéthylène préformé, présentent un inconvénient majeur.

En réalité, malgré de bonnes techniques de fabrication et l'emploi de matériaux performants, ces couches ou serviettes ne peuvent, en aucun cas, empêcher totalement la remontée d'une partie du liquide absorbé, vers les parties en contact avec la peau, avec comme conséquences, rougeurs, érythèmes fessiers et, dans le cas de grabataires alités, la favorisation de formations d'escarres.

Ce procédé, selon l'invention, permet d'apporter un remède à ces inconvénients en laissant réellement et totalement sèches les parties de la couche ou de la serviette périodique en contact avec la peau.

Ce procédé, simple d'application, permet en outre aux fabricants de pouvoir conserver les formats et matériaux choisis pour la réalisation de leurs modèles.

Ce système repose sur deux innovations importantes :
La 1ère : Création de deux pièces ayant comme base un film en polyéthylène (6) ou tous autres matériaux ayant les mêmes caractéristiques de flexibilité et d'impénétrabilité que le polyéthylène, surmontées d'une mince épaisseur absorbante (8) et d'un non tissé anallergique (7) Ces pièces auront la même forme que les zones de stockage des couches et serviettes auxquelles on veut appliquer ce système et sur lesquelles elles seront collées dans leurs contours, laissant libre la zone de miction. Les films servant de base à ces deux pièces empêcheront le liquide accumulé dans les zones de stockage de remonter aux surfaces en contact avec la peau.
La 2ème : Création, par le prolongement du film en polyéthylène, d'ailerons de protection sur les bords transversaux de ces deux pièces limitant la zone de miction. Ces ailerons de sécurité obtenus par double pliage de ce film empêcheront l'humidification des parties à préserver qui se trouvent en contact avec la zone d'écoulement.

Le choix des matériaux absorbants, les épaisseurs des différentes parties, restent à l'appréciation du fabricant, selon les modèles et l'usage auquel ils sont destinés.

Prenons l'exemple d'une couche anatomique de nuit, déjà existante :
Appelons 1, la partie avant (zone périphérique de stockage),
Appelons 2, la partie centrale (zone de miction),
Appelons 3, la partie postérieure (zone périphérique de stockage)

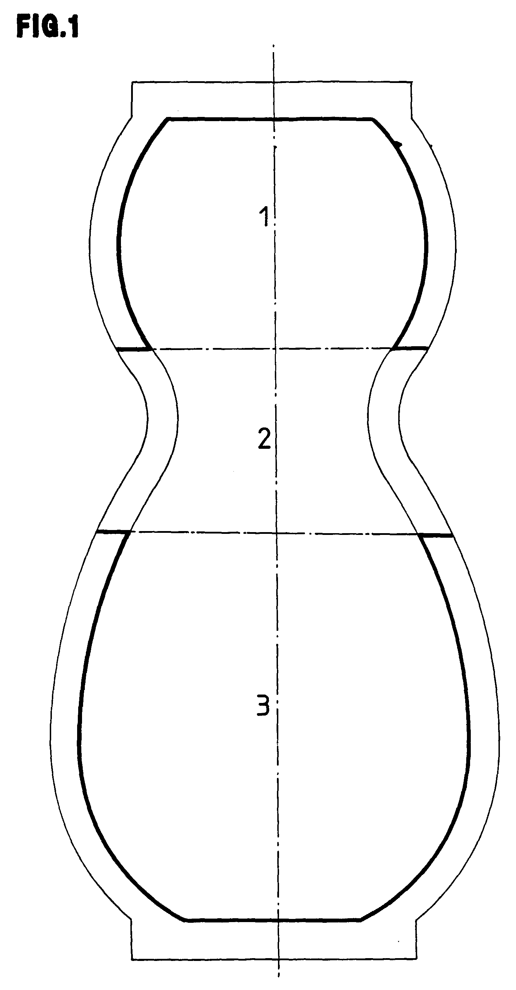

La figure n°1 représente la couche vue de face délimitant les parties 1,2 et 3.

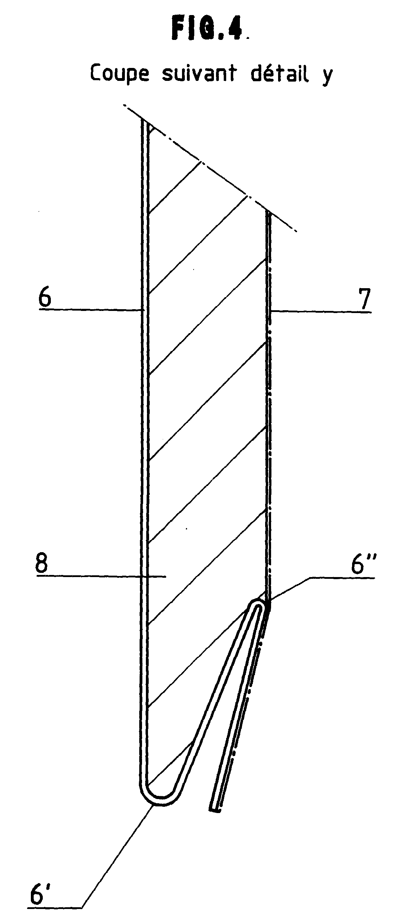

Créons maintenant deux pièces du même format que les zones de stockage de la couche ou serviette à laquelle on veut appliquer ce système, pièces que nous appellerons 4 et 5. Ces pièces seront constituées d'un film en polyéthylène,ou tout autre matériau ayant les mêmes caractéristiques de flexibilité et d'impénétrabilité - Figure n° 4 (6) - et d'une mince couche absorbante - Figure n° 4(8) -.

Sur ces pièces, retournons et collons sur environ 2 centimètres le film en polyéthylène tout le long de la coupe en largeur - Figure n°4 (6') -.

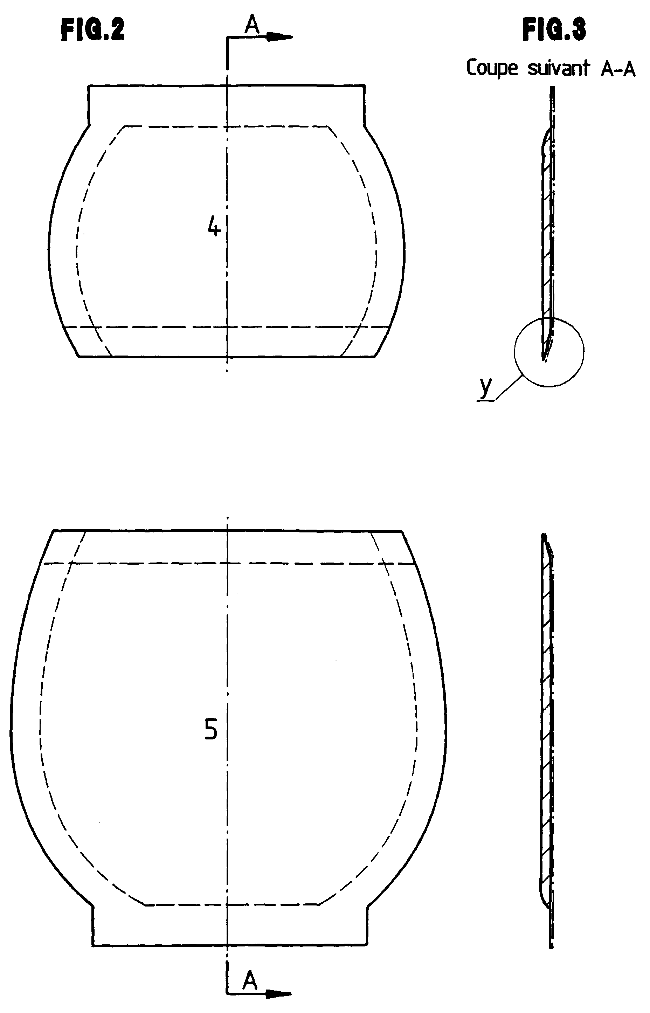

La figure n°2 représente les parties 4 et 5 vues de face.

Plions à nouveau, sans le coller, en sens inverse, le film en polyéthylène de façon à recouvrir exactement la partie collée - Figure n°4 (6'').

Recouvrons d'un voile non tissé anallergique 4 et 5, y compris le repli du film en polyéthylène non collé - Figure n°4 (7) ,
et applatissons les bords des ailerons de protection de telle sorte que, une fois assemblés aux parties 1 et 3, ces bords puissent descendre en pente douce sur la partie n°2.

D'autres modes particuliers de réalisation selon l'invention sont indique's dans les revendications 2-8.

La figure n°3, représente les pièces 4 et 5 en coupe A-A.

La figure n°4, représente le détail des ailerons en coupe.

Collons maintenant sur les contours y compris les coupes transversales au bord de la zone de miction que nous avons appelée n°2, la partie 4 sur la partie 1 de la première couche et la partie 5 sur la partie 3.

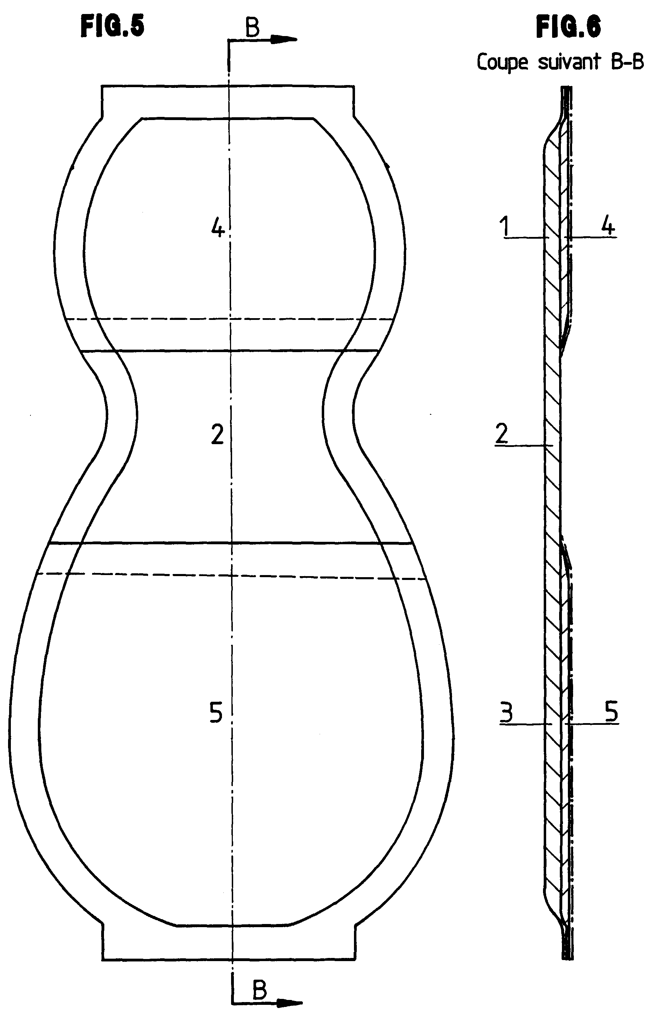

La figure n° 5 représente l'assemblage des parties 4 et 5 sur 1 et 3.

La figure n° 6 représente l'assemblage des différentes parties vu en coupe B-B.

Nous avons ainsi obtenu la pièce définitive, et nous pouvons constater que le liquide qui s'écoulera dans la partie 2 se répandra dans les parties 1 et 3 et le film en polyéthylène, formant la base des pièces 4 et 5 empêchera la remontée du liquide aux couches en contact avec la peau.

Par l'assemblage des différentes parties, nous avons obtenu aux bords des pièces 4 et 5, des ailerons de protection. En réalité nous avons obtenu par collage du 1er pli du film en polyéthylène et le 2ème pliage qui se trouvera, par l'assemblage des différentes pièces, collé uniquement sur les bords, une cavité dans laquelle le liquide non encore absorbé par la partie 2, pourra se répandre sans jamais pouvoir humidifier les parties 4 et 5.

De ce fait, les pièces en contact avec la peau resteront réellement et totalement sèches.

Zone de miction : Ayant ainsi obtenu une étanchéité totale des parties 4 et 5, nous concentrerons toute notre attention à la fabrication de cette partie 2.
- Pour favoriser une évacuation rapide du liquide de l'espace 2 vers les espaces 1 et 3 et pour éviter sa remontée, nous préconisons deux possibilités ou l'association des deux.
   La 1ère possibilité : Placement dans l'espace 2 d'une première couche à haute densité, débordant sur les parties 1 et 3, pressée et percée coniquement uniquement dans la zone de miction.
   La 2ème possibilité : Impression de sillons sur la zone 2, dans le sens de la longueur de la couche sur la totalité de la partie absorbante pour favoriser une plus rapide évacuation du liquide vers les zones de stockage 1 et 3.
- Placer deux barrières verticales de voile non tissé impénétrable de part et d'autre de la couche absorbante, en zone 2, débordant dans les zones de stockage pour éviter tout risque de fuite latérale. Ces aménagements ne sont pas représentés sur nos planches car ils restent à l'appréciation des fabricants et nous ne formulons pas de revendication en ce qui les concerne..

Couches Nuit : Pour les sujets grabataires alités, reposant sur le dos, la seule protection postérieure peut s'avérer suffisante.

Espace d'écoulement entre les deux pièces de protection : Cet espace pourra prendre une forme carrée, rectangulaire, oblongue ou ovale. Cette solution s'est avérée aux essais d'un prix de revient plus élevé et moins fiable. En réalité, un mauvais placement initial ou un déplacement dû aux mouvements pourraient faire se répandre le liquide sur les pièces de protection.

## Revendications

1. Procédé de fabrication d'un système de protection totale applicable à toutes sortes de couches et serviettes périodiques permettant de maintenir réellement et totalement au sec les surfaces en contact de la peau caractérisé en ce que sont créées 2 pièces de protection (4 et 5) de la même forme que les zones de stockage (1 et 3) auxquelles elles sont appliquées et collées sur leur pourtour, les dites pièces sont composées d'un voile anallergique (7) qui se trouve en contact de la peau, d'une mince couche absorbante (8) et d'une base, en film de polyéthylène (6), qui empêche la remontée du liquide accumulé dans les zones de stockage (1 et 3), à la surface des pièces de protection, en ce que par un double pliage (6' et 6'') du prolongement de ce même film, une cavité est obtenue tout le long de la zone de miction en ce que par dans laquelle un excès de liquide, non encore absorbé par la couche de base, peut se répandre sans jamais pouvoir humidifier les bords des pièces de protection.

2. Procédé selon la revendication 1, caractérisé en ce que le film en polyéthylène (6) surmonté de la mince couche absorbante (8), est placé en support des deux pièces de protection.

3. Procédé selon les revendications 1 et 2, caractérisé en ce que les prolongements des films en polyéthylène des pièces de protection sont pliés et collés (6') sur la moitié de la largeur de leur pli, sur leur couche absorbante, tout le long de la coupe en largeur aux limites de l'espace de miction.

4. Procédé selon la revendication 3, caractérisé en ce que les moitiés des plis des films en polyéthylène sont repliés en sens inverse (6'') sur les parties collées de ces mêmes films.

5. Procédé selon la revendication 4, caractérisé en ce que les replis sont recouverts, avec l'ensemble des pièces de protection, d'un non tissé anallergique (7).

6. Procédé selon la revendication 5, caractérisé en ce que les pièces de protection seront collées sur leur pourtour sur les zones de stockage, en même temps que les extrémités des replis (6''), laissant libre l'espace central pour la miction.

7. Procédé selon l'une quelconque des revendications précédentes, caractérisé en ce que l'espace resté vide entre les deux pièces de protection peut prendre différentes formes : carrée, rectangulaire, oblongue ou ovale.

8. Procédé selon toutes les revendications précédentes, caractérisé en ce que les deux pièces en polyéthylène placées en support des deux zones de protection et leur prolongement replié pour former les ailerons de protection, peuvent être fabriqués en d'autres matériaux, naturels ou synthétiques, tissés ou non tissés, ayant les mêmes caractéristiques de flexibilité et d'impénétrabilité que le polyéthylène.

## Claims

1. The fabrication process of a full protection system aplicable to all sorts of sanitary diapers and sanitary napking , allowing the surfaces in contact with the skin , to remain very and completely dry, is caracterised by the making of 2 pieces of protection (4+5) of the same shape as the stocking areas (1+3) to which they will be applied and stuck all around the edge , the aforesaid pieces are composed of an nonallergenic veil (7) which will be in contact with the skin , of a thin absorbing nappy (8) and of a base made of polyethylene film (6) which will prevent the liquid that accumulates in the stocking areas from rising to the protection pieces and the novel feature by folding in two (6'+6'') the prolongation of this same film , a cavity is obtained the length of the urination zone , in wich an excess of a liquid not yet absorbed by the base nappy can spread without even dampening the edge of the protection pieces .

2. According to claim 1, the novel feature of the method is the polyethylene film (6) topped with the thin absorbent layer (8) set as a base to the two protection parts.

3. According to claims 1 and 2, the novel feature is the extended polyethylene films of the protection parts are folded and stuck (6') over half width of their folds, on their absorbent layer along the section in width bounding the urination space.

4. According to claim 3, the novel feature is half the polyethylen film folds are folded again the other way round (6'') over the stuck portions of the said films

5. According to claim 4, the novel feature is the folds and the protection parts as a whole are covered with a nonallergenic nonwoven coating (7).

6. According to claim 5, the novel feature is the protection parts will be adhered at their edges to the storage areas, together with the fold ends (6''), leaving the central portion free for urination.

7. According to any of the above claims, the novel feature is the empty space between the two protection parts may be either square, rectangular, oblong or oval.

8. According to all the above claims, the novel feature of the method is the two polyethylene parts topped with the two protection areas and the protection wings resulting from their folded extension can be made of different materials: natural or synthetic, woven or nonwoven, as flexible and impervious as polyethylene.

## Patentansprüche

1. Produktionsverfahren eines Systems zum totalen Schutz bei jeder Sorte Windel oder Damenbinde, das es erlaubt die Flächen tatsächlich und ganz trocken zu halten, die die Haut berühren;
dadurch gekennzeichnet, dass 2 Schutzteile (4 und 5) geschaffen werden, die die gleiche Form haben wie die Stauzonen (1 und 3), worauf sie gelegt und am Rande geklebt werden. Die genannten Teile bestehen aus einem allergiefreien Schleier, der in Kontakt ist mit der Haut, aus einer dünnen Schicht zum Aufsaugen und aus einer Grundschicht, einer Folie aus Polyäthylen, die es verhindert, dass die in den Stauzonen (1 und 3) gespeicherte Flüssigkeit zur Oberfläche der Schutzteile aufsickert, so dass doppeltes Biegen (6' und 6'') der Verlängerung dieser Folie eine Höhle bekommen wird längs der Miktionszone, wo ein Überschuss an Flüssigkeit, die noch nicht aufgesaugt ist, ausfliessen kann, ohne je die Ränder der Schutzteile befeuchten zu können.

2. Verfahren nach Anspruch 1, dadurch gekennzeichnet, dass die Polyäthylenfolie mit der dünnen Aufsaugschicht als Träger der beiden Schutzteile gelegt wird.

3. Verfahren nach den Ansprüchen 1 und 2, dadurch gekennzeichnet, dass die Verlängerung der Polyäthylenfolie der Schutzteile umgestülpt und auf der halben Breite der Falte an der Aufsaugschicht geklebt sind, längs des Breitendurchschnits an den Grenzen der Miktionszone.

4. Verfahren nach Anspruch 3, dadurch gekennzeichnet, dass die Hälften der Polyäthylenfolienfalten in entgegengesetzter Richtung gebogen sind (6'') auf den geklebten Teilen dieser Folien..

5. Verfahren nach Anspruch 4, dadurch gekennzeichnet, dass die Falten sowie die gesamten Schutzteile mit einem nicht gewobenen allergiefreien Stoff (7) bedeckt sind.

6. Verfahren nach Anspruch 5, dadurch gekennzeichnet, dass die Schutzteile auf ihrem ganzen Rande auf den Stauzonen geklebt sind sowie die Enden der Falten (6''), wobei der mittlere Teil zum Harnlassen frei bleibt.

7. Verfahren nach irgendeinem der genannten Ansprüchen, dadurch gekennzeichnet, dass der leer gebliebene Teil zwischen den beiden Schutzteilen verschiedene Formen bekommt: Quadrat, Rechteck, Oval oder Länglich.

8. Verfahren nach allen oben genannten Ansprüchen, dadurch gekennzeichnet, dass die beiden Polyäthylenteile, die die Schutzteile und deren umgestülpte Verlängerung, welche die Schutzflügel bilden, aus anderen natürlichen oder künstlichen gewobenen oder nicht gewobenen Stoffen mit gleichen Biegsamkeits - und Undurchdringlichkeitseigenschaften wie Polyäthylen Hergestellt werden können.
